(19) Europäisches Patentamt
European Patent Office
Office européen des brevets

(11) **EP 3 303 551 B1**

(12) **EUROPEAN PATENT SPECIFICATION**

(45) Date of publication and mention
of the grant of the patent:
**25.10.2023 Bulletin 2023/43**

(21) Application number: **16729825.6**

(22) Date of filing: **03.06.2016**

(51) International Patent Classification (IPC):
*C12N 1/04* *(2006.01)*      *C12N 1/18* *(2006.01)*
*C12G 1/02* *(2006.01)*      *C12G 3/02* *(2019.01)*
*C12N 1/00* *(2006.01)*

(52) Cooperative Patent Classification (CPC):
**C12G 1/02; C12G 3/02; C12N 1/04; C12N 1/18;**
C12G 2200/05

(86) International application number:
**PCT/EP2016/062705**

(87) International publication number:
**WO 2016/193465 (08.12.2016 Gazette 2016/49)**

(54) **A METHOD FOR PRODUCING A FERMENTED BEVERAGE WITH A FROZEN COMPRESSED YEAST**

VERFAHREN ZUR HERSTELLUNG EINES FERMENTIERTEN GETRÄNKS MIT EINER GEFRORENEN KOMPRIMIERTEN HEFE

PROCEDE DE PRODUCTION D'UNE BOISSON FERMENTEE A L'AIDE D'UNE LEVURE COMPRIMEE CONGELEE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2015 EP 15170659**

(43) Date of publication of application:
**11.04.2018 Bulletin 2018/15**

(73) Proprietor: **Chr. Hansen A/S**
**2970 Hoersholm (DK)**

(72) Inventors:
• **BJERRE, Kristine**
**2830 Virum (DK)**
• **SWIEGERS, Jan Hendrik**
**3480 Fredensborg (DK)**
• **BADAKI, Mansour**
**2720 Vanloese (DK)**
• **JENSEN, Katja Sander**
**2650 Hvidovre (DK)**

(56) References cited:
**WO-A1-2011/134952      US-A- 3 615 685**

• **"Technique: Fresh Yeast", , 12 March 2011 (2011-03-12), XP055227553, Retrieved from the Internet: URL:http://www.cookistry.com/2011/03/techn ique-fresh-yeast.html [retrieved on 2015-11-11]**
• **DZIEZAK J D: "YEASTS AND YEAST DERIVATIVES: DEFINITIONS CHARACTERISTICS AND PROCESSING", FOOD TECHNOLOGY, INSTITUTE OF FOOD TECHNOLOGISTS, CHICAGO, IL, US, vol. 41, no. 2, 1 February 1987 (1987-02-01), pages 104-121, XP008178060, ISSN: 0015-6639**
• **REED G ET AL: "TECHNOLOGY OF YEAST USAGE IN WINEMAKING", AMERICAN JOURNAL OF ENOLOGY AND VITICULTURE, AMERICAN SOCIETY FOR ENOLOGY AND VITICULTURE, US , vol. 39, no. 1 1 March 1988 (1988-03-01), pages 83-90, XP008178061, ISSN: 0002-9254 Retrieved from the Internet: URL:http://www.ajevonline.org/content/39/1 /83.abstract [retrieved on 2015-11-11]**

**Description**

FIELD OF THE INVENTION

The present invention relates to a method for producing a fermented beverage comprising the following steps: a) providing a fruit or vegetable substrate for production of the beverage; b) adding frozen compressed yeast with a dry matter content of between 35% and 90% (w/w) to the fruit or vegetable substrate without any re-hydration or re-activating steps; and c) fermenting the substrate with the compressed yeast to obtain the fermented beverage.

BACKGROUND OF THE INVENTION

US 3,615,685 is entitled "Preparation of active dry yeast" and discloses production of baker's yeast including active dry yeast (column 1, lines 1-2). US 3,615,685 does not provide any disclosure of a method for producing a fermented beverage. The only example of use provided is mixture with flour (column 2, lines 43-50), i.e. a completely different matrix.

"Technique: Fresh Yeast", 12 March 2011, XP0055227553, describes that "freezing [of fresh yeast] is a perfectly acceptable method" and also clearly relates to use of yeast for bread baking. It is evident from the document that the freezing had some negative impact on the survival and/or performance of the yeast.

As well-known to the person of ordinary skill in the art of baking, no rehydration or re-activating steps are needed.

In the wine industry the yeast used is normally active dried yeast (ADY) with a dry-matter content of more than 90% (w/w) which needs time-consuming re-hydration and re-activation at specific temperature before inoculation into the wine.

The production of ADY involves several steps where the yeast is first produced in a fermentor, concentrated, filtrated and lastly includes fluid bed drying of the compressed yeast. However, this process means the product needs to go through several steps (re-hydration, re-activation and acclimatization) before it can be used for inoculation.

Because of the drying of the product, the yeast cells are dehydrated and therefore needs to be re-hydrated and then re-activated in suitable media in order to be metabolic active before application to the substrate (e.g. grape juice as in the case of winemaking). This is a very delicate process for the yeast cells and requires a significant amount of time and attention since factors such as temperature, timing and activation media are important to ensure the survival of the cells. Thus, the rehydration of ADY usually demands a large number of skilled man-hours at a commercial winery during the winemaking process.

During the drying process of active dried yeast production, the cellular membranes of the yeast cells lose their permeability barrier function (Roger Boulton et al, 1996, page 124). Therefore, to re-establish this function, it is important to re-hydrate the membranes by adding the yeast in water at 40°C for 20 minutes. Therefore, the yeast re-hydration process typically involves a 20-30 minute rehydration in un-chlorinated water or a water/grape juice mix (2:1) at a temperature of between 35-38°C, followed by the addition of grape juice of the same volume (50:50 juice/water blend) which is kept for another 20-30 minutes before adding it to wine. It is important that the grape juice does not contain any $SO_2$, which could kill the yeast cells during the sensitive process of rehydration (O'Kennedy, 2008). In addition, the rehydration mix must be cooled down with juice after 20 min in water, 5°C at a time. Failure to cool down from the rehydration temperature after 30 minutes can also result in significant cell death (O'Kennedy, 2008). Furthermore, care should be taken to use uncontaminated grape juice for the rehydration protocol as rehydration with contaminated grape juice will result in contamination of all wine fermentation inoculated using the rehydration mixture. Different manufacturers propose variations on this protocol but the critical step is that dehydrated cells need to be exposed to water or a water/juice mixture at specific temperatures, under sanitary conditions and for specific times in order to hydrate properly, thereby avoiding cell death and consequent in-activity. It is not recommended to add the active dried yeast directly to juice since the high sugar concentration, $SO_2$ and other compounds in the grape juice do not allow for optimal rehydration of the yeast. For this reason, none of the wine yeast manufacturers propose the direct inoculation of active dried yeast to grape must.

Active-dried yeast (ADY) loses activity when not optimally rehydrated (Soubeyrand et al. 2006). The incorrect rehydration of ADY can also lead to stuck alcoholic fermentation (O'Kennedy, 2008), i.e. the yeast is not fermenting all of the sugar present in the substrate resulting in a beverage that is too sweet.

Another way to add yeast to a substrate is to use frozen yeast that can be added directly to the substrate (WO2011/134952). In this method the product is frozen cream yeast with a dry matter content below 28% (w/w) that allows for direct inoculation and high survival during direct inoculation. The concentrate is frozen at -50°C. The disadvantage of this method is that it is crucial that the product is frozen at -50°C, distributed by cold chain, and stored at -50°C. Furthermore, the harsh freezing conditions of the concentrate is damaging to the yeast cells affecting their viability. As a result, additives, which stabilize the yeast cells during and after freezing and/or drying, are often added to the liquid concentrate prior to freezing.

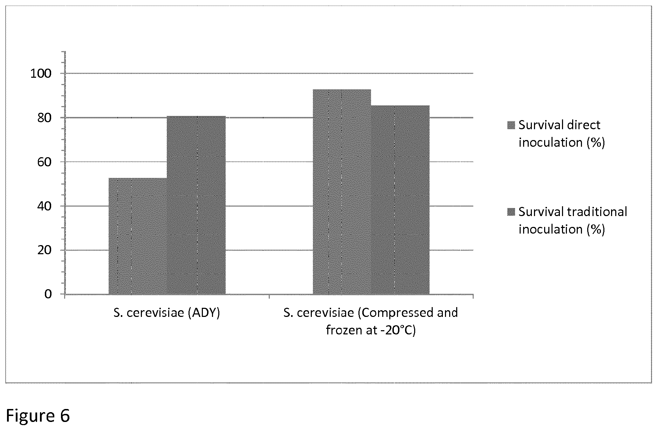

Dziezak J D: "Yeasts and yeast derivatives: Definition characteristics and processing," Food technology, Institute of Food Technologists, Chicago, IL, US, vol. 41, no. 2, 1 February 1987, p. 104-121, XP008178060, discloses ADY. Figure 6 of the present application discloses at left ADY, i.e. the concept disclosed in Dziezak, 1987, and at right the results using frozen compressed yeast according to the present invention. From the results it is evident that no rehydration is necessary for this concept in contrast to the format disclosed in Dziezak, 1987. In summary, neither WO2011/134952 nor Dziezak, 1987 which both relate to the same field as the present invention provides any suggestion or hint to the present invention.

There is an increasing demand from consumers for so-called "clean label" food products, where the number of additives added to the products is limited. The food industry strives to follow the country-specific regulations and recommendations of the food authorities and intergovernmental organizations, such as OIV (International Organisation of Vine and Wine; http://www.oiv.int), EBC (European Brewery Convention) and ASBC (American Society for Brewing Chemists). Furthermore, the use of additives dilutes the concentrate resulting in a lower concentration of yeast cells (colony forming units (CFU) per volume of concentrate) in the final compressed yeast formulation.

## SUMMARY OF THE INVENTION

The improved formulation of yeast for fermentation of beverages by direct inoculation of a fruit or vegetable allows for a higher viability of the yeast in the absence of added additives, such as emulsifiers, oil, water-activity modifying agents and agents added to improve the stability of the yeast cells during freezing, drying and/or cold storage. In addition to the use in fermentation of beverages, it is contemplated that the yeast formulation is well suited for ethanol fermentation by direct inoculation of a carbohydrate-rich liquid substrate.

The present inventors have surprisingly found that compressed yeast, such as wine and brewer's yeast, with a dry matter content of between 35% to 90% (w/w) shows high survival when being frozen despite the relatively high moisture content and in the absence of agents added to improve the stability of the yeast cells during freezing, drying and/or cold storage. In addition, the compressed yeast is ideal for direct inoculation of fruit or vegetable substrates resulting in close to 100% survivability and the compressed yeast can be stored at 4°C for several months under sanitary conditions, e.g. vacuum packed to avoid contamination, or stored for extended periods of time frozen.

## DETAILED DESCRIPTION OF THE INVENTION

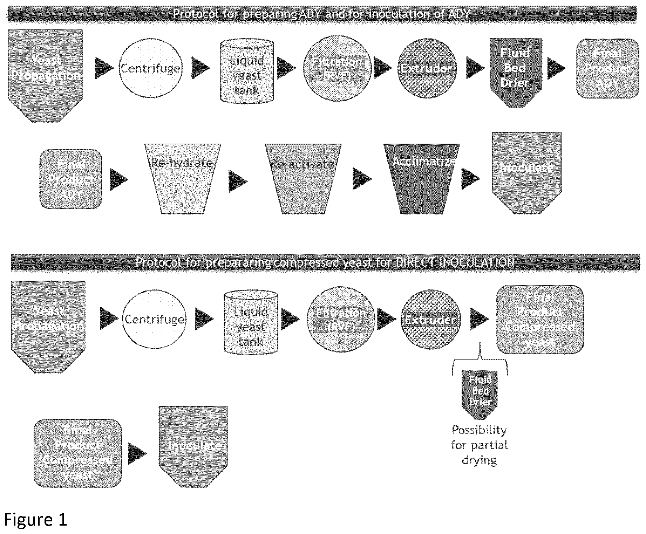

Figure 1 shows the different steps for down-stream processes for production of ADY and inoculation of a substrate with ADY (top) as well as the production of compressed yeast (bottom). As can be seen in Figure 1 (bottom) both the production of compressed yeast as well as the inoculation with compressed yeast is significantly shortened resulting in reduced costs of production.

The compressed yeast for direct inoculation of a fruit or vegetable substrate has a dry matter content of between 35% and 90% (w/w).

In a preferred embodiment, the dry matter content of the compressed yeast is between 45% and 75% (w/w).

The dry matter content of the sample is measured by heating at 105°C +/- 5°C in order to evaporate water content. The sample is measured before and after drying and the below calculations are carried out to get dry-matter content expressed as % (w/w):

The dry matter ($W_{dm}$) content expressed as a percentage of mass or grams per kilogram is calculated using the following equations:

$$W_{dm} = \frac{m_c - m_a}{m_b - m_a} \times f$$

where:

$W_{dm}$ is the dry matter of the sample, in percentages or grams per kilogram;
$m_a$ is the mass of the empty dish or crucible in grams;
$m_b$ is the mass of the dish or crucible containing the sample in grams;
f is a conversion factor, f = 100 for expression of results as a percentage and factor f = 1000 for expression in grams per kilogram.

Values should be rounded to the nearest 0.1% (w/w) or alternatively to the nearest 1 g/kg.

In a preferred embodiment the compressed yeast does not comprise any added additives.

In a preferred embodiment, the viability of the compressed yeast is at least 20% after freezing as calculated based on the concentration of CFU (colony forming units) of the compressed yeast before freezing and the concentration of CFU of the compressed yeast after freezing. Preferably, the viability of the yeast is at least 25%, at least 30%, such as at least 35%, such as at least 40%, such as at least 45%, such as at least 50%, such as at least 55%, such as at least 60%, at least 65%, such as at least 70%, such as at least 75%, such as at least 80%, such as at least 85%, such as at least 90%, such as at least 91%, such as at least 92%, such as at least 93%, such as at least 94%, such as at least 95%, such as at least 96%, such as at least 97%, such as at least 98%, such as at least 99%.

In a preferred embodiment the viability of the compressed yeast is at least 60% after direct inoculation of a fruit or vegetable substrate as calculated based on the concentration of CFU (colony forming units) of the compressed yeast and the concentration of CFU of the inoculated material. Preferably, the viability of the yeast is at least 65%, such as at least 70%, such as at least 75%, such as at least 80%, such as at least 85%, such as at least 90%, such as at least 95%.

In another preferred embodiment the viability of the compressed yeast is at least 80% after storage for five months at a temperature of -20°C followed by direct inoculation of a fruit or vegetable substrate as calculated based on the concentration of CFU (colony forming units) of the compressed yeast before storage and the concentration of CFU of the inoculated material. Preferably, the viability of the yeast is at least 85%, such as at least 90%, such as at least 95%.

In a preferred embodiment, the compressed yeast is frozen at a temperature below 0°C. Preferably, the compressed yeast is frozen at a temperature significantly below 0°C, such as at -5°C, such as at -20°C, such as at -50°C.

In a preferred embodiment, the frozen compressed yeast is frozen in the absence of any additives added to the liquid yeast concentrate in order to stabilize the yeast cells during and after freezing.

In a preferred embodiment, the viability of the frozen compressed yeast is at least 20% after freezing as calculated based on the concentration of CFU (colony forming units) of the compressed yeast before freezing and the concentration of CFU of the compressed yeast after freezing. Preferably, the viability of the yeast is at least 25%, at least 30%, such as at least 35%, such as at least 40%, such as at least 45%, such as at least 50%, such as at least 55%, such as at least 60%, at least 65%, such as at least 70%, such as at least 75%, such as at least 80%, such as at least 85%, such as at least 90%, such as at least 91%, such as at least 92%, such as at least 93%, such as at least 94%, such as at least 95%, such as at least 96%, such as at least 97%, such as at least 98%, such as at least 99%.

The determination of the viability of yeast can be carried out by any suitable method known to the skilled person. In a preferred embodiment the determination of CFU/g and CFU/ml cell counts is performed as set out by OIV in chapter II of the International Oenological Codex, 2013 Issue.

In a preferred embodiment the viability of the frozen compressed yeast is at least 60% after direct inoculation of a fruit or vegetable substrate as calculated based on the concentration of CFU (colony forming units) of the compressed yeast and the concentration of CFU of the inoculated material. Preferably, the viability of the yeast is at least 65%, such as at least 70%, such as at least 75%, such as at least 80%, such as at least 85%, such as at least 90%, such as at least 95%.

In another preferred embodiment the viability of the frozen compressed yeast is at least 80% after storage for five months at a temperature of -20°C followed by direct inoculation of a fruit or vegetable substrate as calculated based on the concentration of CFU (colony forming units) of the compressed yeast before freezing and storage and the concentration of CFU of the inoculated material. Preferably, the viability of the yeast is at least 85%, such as at least 90%, such as at least 95%.

The compressed yeast is preferably present in a concentrated form. In a preferred embodiment the compressed yeast contains at least $10^9$ CFU/g of yeast, such as at least $5 \times 10^9$ CFU/g of yeast, such as at least $10^{10}$ CFU/g of yeast, such as at least $5 \times 10^{10}$ CFU/g of yeast, such as at least $10^{11}$ CFU/g of yeast, such as at least $5 \times 10^{11}$ CFU/g of yeast, such as at least $10^{12}$ CFU/g of yeast.

In a preferred embodiment the compressed yeast is selected from the genera of the group consisting of *Saccharomyces, Kluyveromyces, Lachancea, Torulaspora, Brettanomyces, Pichia, Metschnikowia, Candida, Hanseniaspora, Saccharomycodes, Zygosaccharomyces, Cryptococcus, Issatchenkia, Schizosaccharomyces, Wickerhamomyces* and *Debaryomyces.*

In a preferred embodiment the compressed yeast is a *Saccharomyces* yeast, preferably a *Saccharomyces cerevisiae* yeast

In another preferred embodiment the yeast is a wine yeast or a brewer's yeast, preferably the wine yeast or brewer's yeast is a yeast selected from the group consisting of *Saccharomyces, Kluyveromyces, Lachancea, Torulaspora, Brettanomyces, Pichia* and *Metschnikowia* yeast.

The present invention relates to a method for producing a fermented beverage comprising the following steps:

a) providing a fruit or vegetable substrate for production of the beverage;

b) adding frozen compressed yeast with a dry matter content of between 35% and 90% (w/w) to the fruit or vegetable substrate without any re-hydration or re-activating steps; and

c) fermenting the substrate with the compressed yeast to obtain the fermented beverage.

The fermentation may be carried out under aerobic or anaerobic conditions or it may be carried out under a sequence of aerobic and anaerobic conditions.

In a preferred embodiment the fermented beverage is selected from the group consisting of wine, beer, cider, sake and soft-drinks. Preferably, the fermented beverage is wine.

In another preferred embodiment the fruit or vegetable for production of the beverage is a fruit juice.

Preferably, the fruit juice is grape juice and the fermented beverage is wine.

It is contemplated that the compressed yeast will be equally suitable for use in a similar method of direct inoculation of an aqueous fruit or vegetable substrate for ethanol fermentation under anaerobic conditions. Suitable fruit or vegetable substrates are carbohydrate-rich substrates including but not limited to aqueous solutions based on corn syrup, cane sugar/molasses.

Definitions

The term "cream yeast" herein refers to liquid yeast with a dry matter content of below 28% (w/w) conventionally produced by propagation of yeast in a fermentor followed by concentration by centrifugation.

The term "compressed yeast" refers herein to a yeast with a dry matter content of between 35% and 90% (w/w) conventionally produced by propagation of yeast in a fermentor followed by concentration, filtration, extrusion and optionally partial drying on a drier, such as a fluid bed drier. Thus, the term "partially dried compressed yeast" refers herein to a yeast with a dry matter content of between 45% to 90% (w/w) produced by propagation of yeast in a fermentor followed by concentration, filtration, extrusion and partial drying on a drier, such as a fluid bed drier.

The term "active dried yeast" or "ADY" refers herein to yeast with a dry matter content of more than 90% (w/w) conventionally produced by propagation of yeast in a fermentor followed by concentration, filtration, extrusion and drying on a fluid bed drier.

The term "vegetable" refers herein to any plant. Preferably, the term "vegetable" herein refers to edible plants or edible plant parts.

The term "fruit" refers herein to the edible part of a plant developed from a flower. Fruit may include any accessory tissues of the edible part, such as the skin, peel or pod.

The term "yeast enhancer" herein refers to supplementary nutrients, such as vitamins, nitrogen, phosphate or minerals, added prior to or during propagation of the yeast.

The term "additives" herein refers to food-grade agents which are added to the yeast concentrate at any time point during production of the yeast formulation after propagation of the yeast, e.g. to assist in the extrusion and cutting of the yeast concentrate, e.g. emulsifiers, including, but not limited to, glycerol, glucose, sucrose and trehalose, and oil, to improve the stability of the yeast cells during freezing and/or cold storage, to change the melting point of the frozen yeast formulation, e.g. water-activity modifying agents, etc.

The term "added" when referring to additives herein refers to that the additives are introduced into the yeast concentrate during production of the compressed yeast in an amount efficient to give the desired effect.

BRIEF DESCRIPTION OF THE FIGURES

FIGURE 1 depicts the protocol for preparing ADY and for inoculation of ADY (top) as well as the protocol for preparing compressed yeast for direct inoculation for the preparation of fermented beverages (bottom).

FIGURE 2 shows the survival of *Saccharomyces cerevisiae* yeast either after keeping the concentrate at 4°C or freezing the concentrate at -20°C, at -50°C in the freezer or in liquid nitrogen and storing the yeast for 1 month at the indicated temperature.

FIGURE 3 shows the survival of *Saccharomyces cerevisiae* yeast either after keeping the concentrate at 4°C or freezing the concentrate at -20°C, at -50°C in the freezer or in liquid nitrogen and storing the yeast for 1 month at the indicated temperature followed by direct inoculation in grape must.

FIGURE 4 shows total survival of *Saccharomyces cerevisiae* yeast including both survival after treatment (Figure 3) and survival in direct inoculation (Figure 2).

FIGURE 5 shows the survival of *Saccharomyces cerevisiae* yeast formulated as compressed yeast or as ADY before treatment and after storage for different time periods at the indicated temperatures.

FIGURE 6 shows a comparison between the survival of yeast cells of the *Saccharomyces cerevisiae* ADY yeast and the *Saccharomyces cerevisiae* frozen compressed yeast after direct inoculation and standard inoculation for ADY yeast ("reactivation" of yeast), respectively.

EXAMPLES

**Example 1**

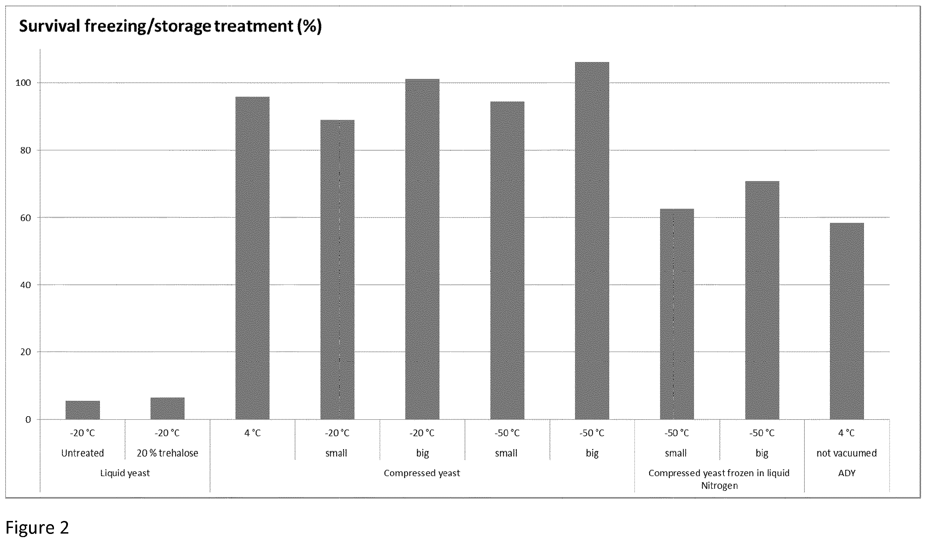

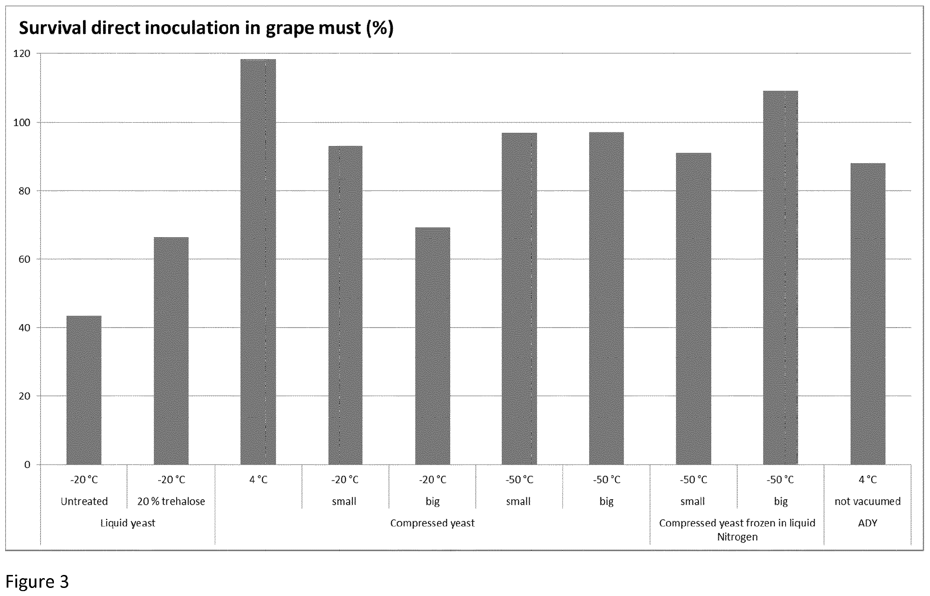

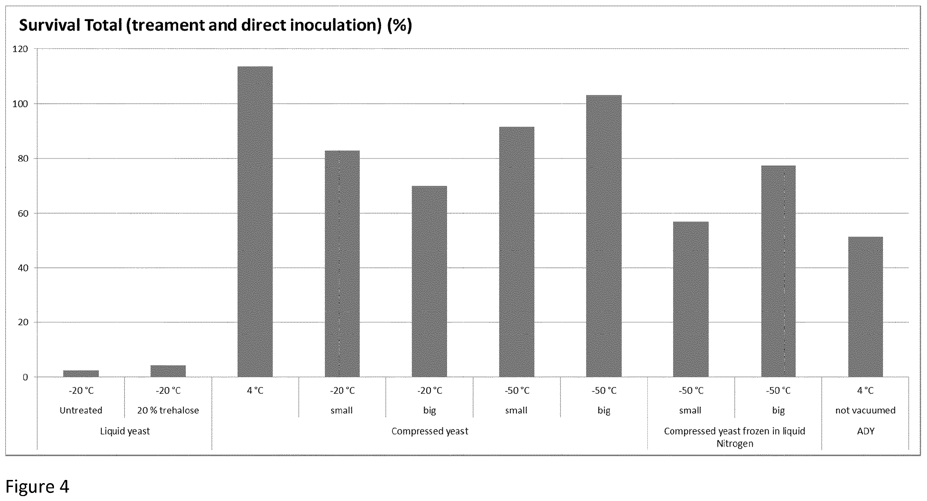

During the production of a conventional *Saccharomyces cerevisiae* strain, samples were taken out at three different stages during production; 1) as liquid (cream) yeast after centrifugation; 2) as compressed yeast (fresh yeast) with a dry matter content of 40% after extrusion; and 3) as active dry yeast (ADY) with a dry matter content of 92% after fluid bed drying:
These samples were analysed according to cell count, survival after either keeping the concentrate at 4°C or freezing the concentrate at -20°C, at -50°C in the freezer or in liquid nitrogen and storing for 1 month at the indicated temperature (Figure 2), survival after either keeping the concentrate at 4°C or freezing the concentrate at -20°C, at - 50°C in the freezer or in liquid nitrogen and storing for 1 month at the indicated temperature followed by direct inoculation in grape must (Figure 3), and total survival in direct inoculation in grape must after either keeping the concentrate at 4°C or freezing the concentrate at -20°C, at -50°C in the freezer or in liquid nitrogen and storing for 1 month at the indicated temperature (Figure 4).

*Survival of treatment and over time*

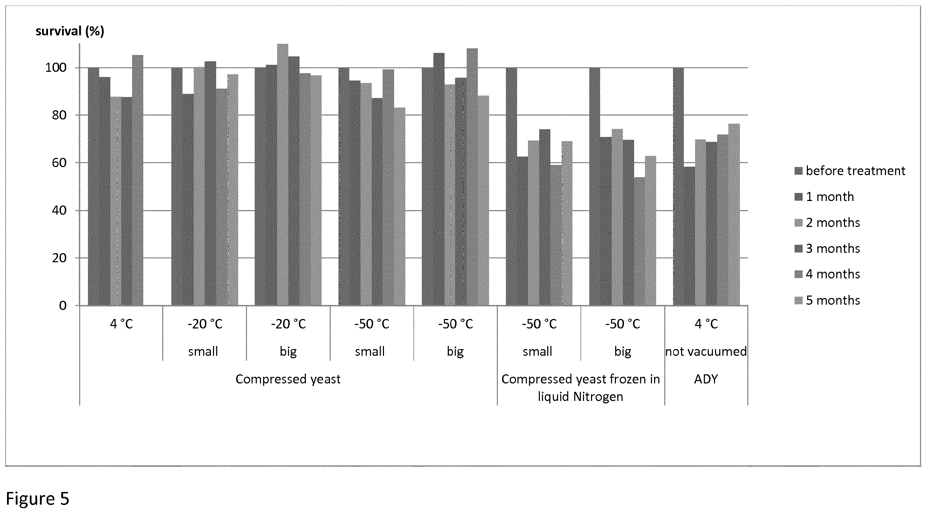

The liquid yeast was frozen with 200 ml sample in a -20°C freezer as untreated or with 20% trehalose. Cell counts were measured before and then again one month after freezing and followed over time.
The compressed yeast was frozen in different sizes and at different temperatures in order to verify the effect of the freezing rate on the survival of yeast cells. The sizes of the samples were 200 ml bottles (big) and 50 ml falcon tubes (small). The temperatures of the freezers were -20°C and -50°C, respectively.
Also the compressed yeast was frozen in liquid nitrogen as pellets (1-2 ml long (small) and as 5 g clumps (big)).
For determination of yeast survival (in percentages), cell counts were measured before freezing and again one month after freezing (Figure 2) and followed over time (Figure 5).

*Survival in direct inoculation*

All samples were also used for direct inoculation after 1 month of storage in order to see the survival when the yeast cells were exposed to water and high osmotic pressure in the grape juice. 1.0 g of samples was added to 200 mL Riesling juice (Table 1). The samples were dissolved by gentle stirring and after approximately 10-15 min. the dilution series was performed and for determination of yeast survival (in percentages), CFU/g was calculated after direct inoculation (Figure 3).

Table 1: Parameters of grape juice used for direct inoculation experiments after 1 month

| Grape juice | Sugar (g/l) | Malic acid (g/l) | TSO$_2$ (mg/l) |
|---|---|---|---|
| Riesling (Germany) | 180 | 6.6 | 0 |

*Comparison of survival by direct inoculation and "reactivation" of the yeast*

Survival of inoculation by use of two different methods was compared. In both methods the inoculation volume was 0.2 g/l. For direct inoculation, the sample was added directly to the Chardonnay juice (Table 2). For standard inoculation, the sample was first rehydrated 1:10 in unchlorinated water for approximately half an hour. Un-sulphured grape juice was then added to the water in the ratio (1:3) and the suspension was then left to activate for approximately another 20 minutes. The final activated suspension was then added to the juice to reach a final inoculation level of 0.2 g/l. All inoculations were performed in duplicates.
CFU/g of the products were calculated before inoculation and after inoculation and the survival was then calculated as percentages (Figure 6).

Table 2: Parameters of grape juice used for direct inoculation experiments after 8 months

| Grape juice | Sugar (g/l) | Malic acid (g/l) | $TSO_2$ (mg/l) |
|---|---|---|---|
| Chardonnay (Germany) | 180 | 6.7 | 0 |

*Determination of colony forming units (CFU)*

For determination of CFU/g and CFU/ml cell counts from samples taken from either inoculated grape juice or yeast dissolved in peptone water were performed by pour-plating on YGC media (prepared as set out by OIV in appendix VI of the International Oenological Codex, 2013 Issue). 1 ml of the sample from the dilution series (peptone water) was added to the plate and on top of that the liquid YGC agar (45°C) was poured over the sample and mixed. After setting, the plates were incubated at 30°C for 2-3 days. Determinations of CFU were in all cases performed in triplicates.

*Conclusion:*

The results show that the survival of the yeast in direct inoculation (Figure 2) is very high for especially compressed yeast stored at 4°C and compressed yeast frozen at -50°C or in liquid nitrogen.

The survival of the yeast after different treatments and storage of the samples for 1 month at this temperature shows high variations (Figure 3). Surprisingly, the compressed yeast with high water content shows close to 100% survival when frozen at -20°C and -50°C. When the compressed yeast is frozen in liquid nitrogen the survival (>60%) is also surprisingly high. The liquid yeast shows very low survival during freezing and the ADY also loose a high percentage of CFU when stored for 1 month at 4°C.

The overall survival shows that the compressed yeast, even when frozen, shows the highest survival (Figure 4). The survival of the compressed yeast frozen at -50°C and used in direct inoculation is close to 100%.

Stability of the different formats is measured over 5 months and results are shown in (Figure 5). The cell counts of the compressed yeast samples are very stable even though the temperature has not been constant as the samples have been taken out several times.

The compressed yeast stored at 4°C was only followed for 4 months as the sample was not packed in a proper sanitary way to be kept for a longer period of time.

The active dry yeast (ADY) products show lower survival when directly inoculated compared to when inoculated after rehydration and reactivation (Figure 6). When used for direct inoculation the ADY shows an average survival of 53% and when inoculated after rehydration and reactivation the survival is 81% on average. For the frozen compressed yeast the survival in direct inoculation is 93% and survival when inoculated according to the "standard" procedure is 86% which is also higher than the average of the ADY products.

REFERENCES

Roger Boulton, Vernon Singleton, Linda Bisson and Ralph Kunkee. 1996. Principles and Practices of Winemaking. pp. 124.

Karien O'Kennedy. (2008). How to avoid stuck fermentations, The Australian & New Zealand Grapegrower & Winemaker. November, Issue 538, 103-105.

Virginie Soubeyrand, Anne Julien and Jean-Marie Sablayrolles (2006) Rehydration Protocols for Active Dry Wine Yeasts and the Search for Early Indicators of Yeast Activity American Journal of Enology and Viticulture. 57(4)474-480.

**Claims**

1. A method for producing a fermented beverage comprising the steps:

a) providing a fruit or vegetable substrate for production of the beverage;
b) directly inoculating the substrate with a compressed yeast, wherein the compressed yeast is a frozen compressed yeast with a dry matter content of between 35% and 90% (w/w); and

c) fermenting the substrate with the compressed yeast to obtain the fermented beverage.

2. The method according to claim 1, wherein the compressed yeast is a frozen compressed yeast, wherein the dry matter content is between 45% and 75% (w/w).

3. The method according to claim 1 or 2, wherein the compressed yeast does not comprise any added additives.

4. The method according to any of the preceding claims, wherein the compressed yeast is frozen in the absence of any added additives added to the liquid yeast concentrate in order to stabilize the yeast cells during and after freezing.

5. The method according to any of the preceding claims, wherein the viability of the yeast is at least 20% after freezing as calculated based on the concentration of CFU (colony forming units) of the compressed yeast before freezing and the concentration of CFU of the compressed yeast after freezing.

6. The method according to any of the preceding claims, wherein the viability of the yeast is at least 60% after direct inoculation of a fruit or vegetable substrate as calculated based on the concentration of CFU (colony forming units) of the compressed yeast and the concentration of CFU of the inoculated material.

7. The method according to any of the preceding claims, wherein the viability of the yeast is at least 80% after storage for five months at a temperature of -20°C followed by direct inoculation of a fruit or vegetable substrate as calculated based on the concentration of CFU (colony forming units) of the compressed yeast before freezing and the concentration of CFU of the inoculated material.

8. The method according to any of the preceding claims, wherein the yeast is selected from the genera of the group consisting of *Saccharomyces, Kluyveromyces, Lachancea, Torulaspora, Brettanomyces, Pichia, Metschnikowia, Candida, Hanseniaspora, Saccharomycodes, Zygosaccharomyces, Cryptococcus, Issatchenkia, Schizosaccharomyces, Wickerhamomyces* and *Debaryomyces.*

9. The method according to claim 8, wherein the yeast is a *Saccharomyces* yeast.

10. The method according to any of the preceding claims, wherein the fermented beverage is selected from the group consisting of wine, beer, cider, sake and soft-drinks.

11. The method according to claim 10, wherein the substrate is a fruit juice.

12. The method according to claim 11, wherein the fruit juice is grape juice and the fermented beverage is wine.

**Patentansprüche**

1. Verfahren zum Herstellen eines fermentierten Getränks umfassend die Schritte:

a) Bereitstellen eines Obst- oder Gemüsesubstrats zur Herstellung des Getränks;
b) direktes Inokulieren des Substrats mit einer komprimierten Hefe, wobei die komprimierte Hefe eine gefrorene komprimierte Hefe mit einem Trockenmassegehalt zwischen 35 % und 90 % (Gew./Gew.) ist; und
c) Fermentieren des Substrats mit der komprimierten Hefe, um das fermentierte Getränk zu erhalten.

2. Verfahren nach Anspruch 1, wobei die komprimierte Hefe eine gefrorene, komprimierte Hefe ist, wobei der Trockenmassegehalt zwischen 45 % und 75 % ( Gew./Gew.) liegt.

3. Verfahren nach Anspruch 1 oder 2, wobei die komprimierte Hefe keine zugesetzten Zusatzstoffe umfasst.

4. Verfahren nach einem der vorhergehenden Ansprüche, wobei die komprimierte Hefe gefroren wird, ohne dass dem flüssigen Hefekonzentrat irgendwelche Zusatzstoffe zugesetzt werden, um die Hefezellen während und nach dem Einfrieren zu stabilisieren.

5. Verfahren nach einem der vorhergehenden Ansprüche, wobei die Viabilität der Hefe mindestens 20 % nach dem Einfrieren beträgt, berechnet basierend auf der Konzentration von KBE (koloniebildenden Einheiten) der komprimierten Hefe vor dem Einfrieren und der Konzentration von KBE der komprimierten Hefe nach dem Einfrieren.

6. Verfahren nach einem der vorhergehenden Ansprüche, wobei die Viabilität der Hefe mindestens 60 % nach direkter Inokulation eines Obst- oder Gemüsesubstrats beträgt, berechnet basierend auf der Konzentration von KBE (koloniebildenden Einheiten) der komprimierten Hefe und der Konzentration von KBE des inokulierten Materials.

7. Verfahren nach einem der vorhergehenden Ansprüche, wobei die Viabilität der Hefe mindestens 80 % nach fünfmonatiger Lagerung bei einer Temperatur von -20 °C und anschließender direkter Inokulation eines Obst- oder Gemüsesubstrats beträgt, berechnet basierend auf der Konzentration von KBE (koloniebildenden Einheiten) der komprimierten Hefe vor dem Einfrieren und der Konzentration von KBE des inokulierten Materials.

8. Verfahren nach einem der vorhergehenden Ansprüche, wobei die Hefe aus den Gattungen der Gruppe bestehend aus *Saccharomyces, Kluyveromyces, Lachancea, Torulaspora, Brettanomyces, Pichia, Metschnikowia, Candida, Hanseniaspora, Saccharomycodes, Zygosaccharomyces, Cryptococcus, Issatchenkia, Schizosaccharomyces, Wickerhamomyces* und *Debaryomyces* ausgewählt wird.

9. Verfahren nach Anspruch 8, wobei die Hefe eine *Saccharomyces-Hefe* ist.

10. Verfahren nach einem der vorhergehenden Ansprüche, wobei das fermentierte Getränk aus der Gruppe bestehend aus Wein, Bier, Most, Sake und Erfrischungsgetränken ausgewählt wird.

11. Verfahren nach Anspruch 10, wobei das Substrat ein Fruchtsaft ist.

12. Verfahren nach Anspruch 11, wobei der Fruchtsaft Traubensaft und das fermentierte Getränk Wein ist.

**Revendications**

1. Procédé de production d'une boisson fermentée comprenant les étapes de :

   a) fourniture d'un substrat de fruit ou de légume pour la production de la boisson ;
   b) inoculation du substrat directement avec une levure comprimée, dans lequel la levure comprimée est une levure comprimée congelée ayant une teneur en matière sèche comprise entre 35 % et 90 % (p/p) ; et
   c) fermentation du substrat avec la levure comprimée pour obtenir la boisson fermentée.

2. Procédé selon la revendication 1, dans lequel la levure comprimée est une levure comprimée congelée, dans lequel la teneur en matière sèche est comprise entre 45 % et 75 % (p/p).

3. Procédé selon la revendication 1 ou 2, dans lequel la levure comprimée ne comprend aucun additif ajouté.

4. Procédé selon l'une quelconque des revendications précédentes, dans lequel la levure comprimée est congelée en l'absence de tout additif ajouté au concentré de levure liquide afin de stabiliser les cellules de levure pendant et après la congélation.

5. Procédé selon l'une quelconque des revendications précédentes, dans lequel la viabilité de la levure est d'au moins 20 % après congélation telle que calculée sur la base de la concentration en UFC (unités formant colonie) de la levure comprimée avant congélation et de la concentration en UFC de la levure comprimée après congélation.

6. Procédé selon l'une quelconque des revendications précédentes, dans lequel la viabilité de la levure est d'au moins 60 % après inoculation directe d'un substrat de fruit ou de légume telle que calculée sur la base de la concentration en UFC (unités formant colonie) de la levure comprimée et la concentration en UFC du matériau inoculé.

7. Procédé selon l'une quelconque des revendications précédentes, dans lequel la viabilité de la levure est d'au moins 80 % après un stockage pendant cinq mois à une température de -20 °C suivi d'une inoculation directe d'un substrat de fruit ou de légume telle que calculée sur la base de la concentration en UFC (unités formant colonie) de la levure comprimée et la concentration en UFC du matériau inoculé.

8. Procédé selon l'une quelconque des revendications précédentes, dans lequel la levure est choisie parmi les genres du groupe constitué de *Saccharomyces, Kluyveromyces, Lachancea, Torulaspora, Brettanomyces, Pichia, Metschnikowia, Candida, Hanseniaspora, Saccharomycodes, Zygosaccharomyces, Cryptococcus, Issatchenkia, Schizosaccharomyces, Wickerhamomyces* et *Debaryomyces.*

9.  Procédé selon la revendication 8, dans lequel la levure est une levure *Saccharomyces.*

10. Procédé selon l'une quelconque des revendications précédentes, dans lequel la boisson fermentée est choisie dans le groupe constitué du vin, de la bière, du cidre, du saké et des boissons sans alcool.

11. Procédé selon la revendication 10, dans lequel le substrat est un jus de fruits.

12. Procédé selon la revendication 11, dans lequel le jus de fruits est du jus de raisin et la boisson fermentée est du vin.

Figure 1

Figure 2

Figure 3

EP 3 303 551 B1

**Survival Total (treament and direct inoculation) (%)**

Figure 4

Figure 5

Figure 6

**EP 3 303 551 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3615685 A

- WO 2011134952 A


**Non-patent literature cited in the description**

- *Technique: Fresh Yeast,* 12 March 2011
- Yeasts and yeast derivatives: Definition characteristics and processing. **DZIEZAK J D.** Food technology. Institute of Food Technologists, 01 February 1987, vol. 41, 104-121
- **ROGER BOULTON ; VERNON SINGLETON ; LINDA BISSON ; RALPH KUNKEE.** *Principles and Practices of Winemaking,* 1996, 124

- **KARIEN O'KENNEDY.** How to avoid stuck fermentations. *The Australian & New Zealand Grapegrower & Winemaker,* November 2008, (538), 103-105
- **VIRGINIE SOUBEYRAND ; ANNE JULIEN ; JEAN-MARIE SABLAYROLLES.** *Rehydration Protocols for Active Dry Wine Yeasts and the Search for Early Indicators of Yeast Activity American Journal of Enolology and Viticulture,* 2006, vol. 57 (4), 474-480